(19) Europäisches Patentamt
European Patent Office
Office européen des brevets

(11) EP 2 916 146 A1

(12) EUROPÄISCHE PATENTANMELDUNG

(43) Veröffentlichungstag:
09.09.2015 Patentblatt 2015/37

(51) Int Cl.:
*G01T 1/24* (2006.01)          *G01T 1/40* (2006.01)

(21) Anmeldenummer: 15156494.5

(22) Anmeldetag: 25.02.2015

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: 05.03.2014 DE 102014204042

(71) Anmelder: **Siemens Aktiengesellschaft**
**80333 München (DE)**

(72) Erfinder:
• **Göderer, Edgar**
  **91301 Forchheim (DE)**
• **Kreisler, Björn**
  **91353 Hausen (DE)**

(54) **VERFAHREN ZUR ANSTEUERUNG EINES RÖNTGENDETEKTORS UND ZUGEHÖRIGE STEUEREINHEIT**

(57) Es werden ein Verfahren zur Ansteuerung eines Röntgendetektors (1), der eine röntgensensitive Sensorschicht (2) sowie eine Anordnung von mit der Sensorschicht (2) rückseitig verbundenen Pixelelektroden (7) umfasst, sowie eine zugehörige Steuereinheit (15) angegeben. Verfahrensgemäß wird an jede der Pixelelektroden (7) eine individuell eingestellte Depletionsspannung ($U_D$) angelegt. Die an verschiedene Pixelelektroden (7) angelegten Depletionsspannungen ($U_D$) werden dem Betrag nach derart unterschiedlich gewählt, dass die den Pixelelektroden (7) jeweils zugeordneten effektiven Pixelgrößen (G) aneinander angeglichen werden.

FIG 1

EP 2 916 146 A1

**EP 2 916 146 A1**

**Beschreibung**

Die Erfindung bezieht sich auf ein Verfahren zur Ansteuerung eines Röntgendetektors. Die Erfindung bezieht sich weiterhin auf eine Steuereinheit zur Durchführung des Verfahrens.

Eine zentrale Grundanforderung der Röntgenbildgebung, insbesondere im Bereich der Computertomographie (CT), ist die Signalstabilität und -reproduzierbarkeit. Im Idealfall soll der Röntgendetektor in Antwort auf die gleiche Eingangsstrahlung immer das gleiche Messergebnis liefern. In der Realität wird die Detektorantwort jedoch von etlichen Faktoren beeinflusst. Langfristige Einflussgrößen sind hier insbesondere Altersprozesse, die Strahlendosis der einfallenden Röntgenstrahlung sowie schwankende Betriebsbedingungen infolge von An-/Ausschaltzyklen. Einer kurzfristigen (vorübergehenden) Beeinflussung unterliegt ein Röntgendetektor insbesondere durch die Bestrahlungshistorie sowie infolge von Temperaturänderungen (die wiederum strahlungsbedingt sein können). In der Computertomographie werden Schwankungen der Detektorantwort (bei konstanter einfallender Röntgenstrahlung) zumeist als "Drift" bezeichnet.

Insbesondere in der Computertomographie werden in zunehmendem Maße sogenannte direktwandelnde, quantenzählende Röntgendetektoren eingesetzt. Ein solcher Röntgendetektor weist üblicherweise eine röntgensensitive Sensorschicht auf, in der durch einfallende Röntgenquanten Elektronen-Loch-Paare erzeugt werden. Durch Anlegen einer sogenannten Depletionsspannung werden die strahlungsinduzierten Elektronen-Loch-Paare getrennt. Hierzu ist auf einer - der Strahlungsquelle in der Regel zugewandten - Vorderseite der Sensorschicht üblicherweise eine sich über die gesamte Detektorfläche erstreckende Steuerelektrode aufgebracht, die auf ein negatives elektrisches Potential gelegt ist. Auf der von der Vorderseite abgewandten Rückseite der Sensorschicht ist eine Anordnung von abgrenzten Elektroden angeordnet. Diese rückseitigen Elektroden, von denen jede einen Bildpunkt des aufzunehmenden Röntgenbildes erzeugt, und die daher im Folgenden als "Pixelelektroden" bezeichnet sind, sind in der Regel auf Massenpotential gelegt.

Unter Wirkung der - typischerweise in der Größenordnung von 1000 Volt liegenden - Depletionsspannung driften die durch ein Röntgenquant "ausgeschlagenen" Elektronen zu einer der Pixelelektroden und lösen dort einen Strompuls aus. Der Begriff "Depletion" bezeichnet in diesem Sinne die Entziehung der strahlungsinduzierten freien Elektronen aus der Sensorschicht. Die an den einzelnen Pixelelektroden erzeugten Strompulse werden von einer nachgeschalteten Auswerteelektronik als Zählereignis erfasst. Die Auswerteelektronik unterzieht die Spitzenstromstärke der erfassten Strompulse in der Regel einer Schwellwertanalyse, um echte, also strahlungsinduzierte Zählereignisse von sonstigen Störströmen zu unterscheiden. Bisweilen führt die Steuerelektronik hierbei einen abgestuften Schwellwertvergleich durch, im Zuge dessen die Strompulse nach Maßgabe der Spitzenstromstärke in unterschiedlichen Kanälen gezählt werden. Da die Spitzenstromstärke (Pulshöhe) der strahlungsinduzierten Strompulse von der Quantenenergie der erfassten Röntgenquanten abhängt, ermöglicht dies eine spektrale (also nach der Frequenz oder äquivalenterweise der Quantenenergie aufgelöste) Erfassung der detektierten Röntgenstrahlung.

Bei Röntgendetektoren, insbesondere Röntgendetektoren der vorstehend beschriebenen Art, hat die Drift regelmäßig eine räumliche Verteilung über die Sensorfläche. Die Detektorantwort ist also - bei räumlicher Homogenität der einfallenden Röntgenstrahlung - räumlich inhomogen. Der statische Anteil dieser räumlichen Inhomogenität wird in der herkömmlichen Computertomographie meist durch Aufnahme eines Referenzbilds unter homogener Bestrahlung und durch Division mit der relativen Zählrate kompensiert. Dieses Kompensationsverfahren lässt allerdings die Ursachen für die Inhomogenität der Detektorantwort unberührt. Zudem ist dieses Verfahren nicht zur Kompensation der kurzfristigen Drift (also der kurzfristigen zeitlichen Änderung der Detektorantwort) geeignet. Diese lässt sich vielmehr derzeit nur durch verbesserte Sensormaterialien oder durch Konditionierung des Sensormaterials (beispielsweise mittels Infrarotstrahlung) mindern. Diese Maßnahmen sind jedoch nur begrenzt wirksam und häufig mit einer erheblichen Steigerung des Herstellungs- und Betriebsaufwandes verbunden.

Der Erfindung liegt die Aufgabe zugrunde, die Drift bei einem Röntgendetektor, insbesondere einem direktwandelnden, quantenzählenden Röntgendetektor auf einfach umsetzbare, dennoch aber effektive Weise zu eliminieren oder zumindest zu reduzieren.

Bezüglich eines Verfahrens zur Ansteuerung eines Röntgendetektors wird diese Aufgabe erfindungsgemäß gelöst durch die Merkmale des Anspruchs 1. Bezüglich einer Steuereinheit zur Durchführung des Verfahrens wird die Aufgabe erfindungsgemäß gelöst durch die Merkmale des Anspruchs 8. Vorteilhafte und teils für sich gesehen erfinderische Ausgestaltungsformen und Weiterentwicklungen der Erfindung sind in den Unteransprüchen und der nachfolgenden Beschreibung dargelegt.

Die Erfindung geht von einem Röntgendetektor aus, der eine röntgensensitive Sensorschicht sowie eine Anordnung von Pixelelektroden umfasst, wobei der Sensorschicht strahlungsinduzierte Ladungsträger unter Wirkung einer Depletionsspannung entzogen werden. Bei dem Röntgendetektor handelt es sich vorzugsweise um einen direktwandelnden, quantenzählenden Röntgendetektor der vorstehend beschriebenen Art. Grundsätzlich ist die Erfindung aber auch bei anderen Detektortypen anwendbar.

Verfahrensgemäß wird die an die Pixelelektroden angelegte Depletionsspannung nicht für alle Pixelelektroden einheitlich eingestellt. Vielmehr wird diese Depletionsspannung für jede der Pixelelektroden individuell eingestellt. Die an die verschiedenen Pixelelektroden angelegten Depletionsspannungen werden dabei derart verschieden gewählt, dass die den Pixelelektroden jeweils zugeordneten effektiven Pixelgrößen einander angeglichen werden, dass also die effektive Pixelgröße für alle Pixelelektroden des Röntgendetektors zumindest näherungsweise den gleichen Wert hat.

Als die einer Pixelelektrode zugeordnete "effektive Pixelgröße" (nachfolgend kurz auch als "Pixelgröße" bezeichnet) wird die Querschnittsfläche desjenigen Volumens der Sensorschicht bezeichnet, in dem die Absorption eines Röntgenquants zu einem Strompuls auf der betreffenden Pixelelektrode führt. Salopp gesagt bezeichnet die effektive Pixelgröße somit den Einflussbereich einer Pixelelektrode innerhalb der Sensorschicht. Die effektive Pixelgröße übersteigt hierbei in der Regel den Flächeninhalt der zugehörigen Pixelelektrode, zumal sich der Einflussbereich einer jeden Pixelelektrode mit dem von dieser ausgehenden elektrischem Feld in die zwischen den Pixelelektroden gebildeten Zwischenräume hinein erstreckt.

Die Depletionsspannungen werden vorzugsweise an den Pixelelektroden eingestellt, nämlich durch individuelle Einstellung des elektrischen (Betriebs-)Potentials der jeweiligen Pixelelektrode. Die Pixelelektroden des Röntgendetektors werden somit gezielt auf - relativ zueinander - verschiedene Betriebspotentiale gelegt. Die von den Pixelelektroden abgewandte Vorderseite des Röntgendetektors wird dagegen vorzugsweise - analog zu herkömmlichen Röntgendetektoren - durch eine die Sensorfläche überspannende Steuerelektrode auf einheitlichem elektrischem Potential gehalten.

Die Erfindung geht von der Überlegung aus, dass sich bei Röntgendetektoren, insbesondere direktwandelnden, quantenzählenden Röntgendetektoren, zwei Ursachen für die auftretende Drift unterscheiden lassen, nämlich einerseits die sogenannte "spektrale Stauchung" (Spectral Squeezing) und andererseits die sogenannte "Pixelgrößendrift".

Als "spektrale Stauchung" wird eine Veränderung der Form des bei Absorption eines Röntgenquants erzeugten Strompulses bezeichnet. So wird durch die Absorption eines Röntgenquants mit gegebener Quantenenergie zwar stets die gleiche Ladungsmenge freigesetzt. Je nach Absorptionsort und der Verteilung der Driftgeschwindigkeit der Ladungsträger kann der durch diese Ladungsträger erzeugte Strompuls allerdings eine variierende Breite (d.h. zeitliche Dauer) und Höhe (Spitzenstromstärke) aufweisen. Die spektrale Stauchung beruht hierbei auf einer Veränderung des Feldstärkenverlaufs im Bereich des sogenannten "Weighting Fields", d.h. des Felds in dem in der Nähe der Pixelelektrode liegenden Bereich der Sensorschicht, in der die erzeugte Signalladung den Hauptteil ihres Signales influenziert. Sinkt hier die Feldstärke, so sinkt auch die Driftgeschwindigkeit der Ladungsträger, so dass die influenzierten Pulse breiter werden. Bei einem auf eine gewisse Spitzenstromstärke kalibrierten Schwellwertvergleich, wie er durch die Auswerteelektronik eines quantenzählenden Röntgendetektors üblicherweise durchgeführt wird, führt die Verbreitung der Strompulse regelmäßig dazu, dass weniger Pulse als Zählereignisse gezählt werden.

"Pixelgrößendrift" bezeichnet andererseits eine Veränderung der effektiven Pixelgröße. Die Ursache der Pixelgrößendrift liegt hierbei in einer Änderung der elektrischen Feldkonfiguration innerhalb der Sensorschicht, die den Einflussbereich der Pixelelektrode (also das Volumen der Sensorschicht, aus dem die entstehende Ladung der Pixelelektrode zufließt) ändert. Da die Sensorfläche insgesamt unveränderlich ist, geht hierbei eine Zunahme der effektiven Pixelgröße einer Pixelelektrode stets zu Lasten einer oder mehrerer benachbarter Pixelelektroden. Ursachen für die Pixelgrößendrift können beispielsweise eine elektrische Aufladung von Versetzungszonen oder Störstellen im Material der Sensorschicht sein. Im Gegensatz zu der spektralen Stauchung wird durch Pixelgrößendrift die Rate der in der betreffenden Pixelelektrode erzeugten Strompulse verändert, während die Spitzenstromstärke der einzelnen Strompulse von der Pixelgrößendrift unbeeinflusst bleibt.

Bekanntermaßen kann nun durch die räumliche Variation der Depletionsspannung die Ursache für die Pixelgrößendrift beseitigt werden. Die Pixelgrößendrift wird somit nicht lediglich nachträglich durch Signalbearbeitung kompensiert (was stets ein gewisses Fehlerrisiko beinhaltet). Vielmehr wird durch das erfindungsgemäße Verfahren die Pixelgrößendrift bereits an ihrer Entstehung gehindert. Durch die Beseitigung dieses Driftanteils wird die Bildqualität des Röntgendetektors wesentlich verbessert.

In vorteilhafter Ausführung des Verfahrens werden die an die Pixelelektroden jeweils angelegten Depletionsspannungen nicht nur örtlich (also von Pixelelektrode zu Pixelelektrode) variiert. Vielmehr wird die an jede der Pixelelektroden angelegte Depletionsspannung vorzugsweise auch zeitlich variiert. Als Eingangsgröße, nach Maßgabe welcher die Depletionsspannungen in zeitlicher Hinsicht variiert werden, wird hierbei die mittels des Röntgendetektors erfasste Röntgenstrahlung herangezogen. Dieser Vorgehensweise liegt die Erkenntnis zugrunde, dass die Pixelgrößendrift maßgeblich von der Bestrahlungshistorie des Röntgendetektors abhängt. So führt die Bestrahlung des Röntgendetektors mit intensiver Röntgenstrahlung bekanntermaßen dazu, dass sich Inhomogenitäten in der räumlichen Verteilung der effektiven Pixelgröße herausbilden oder verstärken, wobei diese Inhomogenitäten die auslösende Bestrahlungsphase überdauern und nur allmählich abklingen.

In einer Variante des Verfahrens wird hierbei die (räumlich und/oder zeitlich) gemittelte Intensität der erfassten Röntgenstrahlung als Eingangsgröße für die zeitliche Variation der Depletionsspannungen herangezogen. Es ist grundsätzlich im Rahmen der Erfindung denkbar, dass lediglich der aktuelle Wert der Röntgenintensität berücksichtigt wird. Für eine präzisere Anpassung der Depletionsspannungen wird vorzugsweise aber auch der zeitliche Verlauf der Röntgenintensität betrachtet, beispielsweise indem aus mehreren, zeitlich aufeinander folgend bestimmten Werten der gemittelten Röntgenintensität ein - vorzugsweise gewichteter - Mittelwert oder Trend bestimmt und als Eingangsgröße für die zeitliche Variation der Depletionsspannungen herangezogen wird.

Alternativ hierzu werden anstelle der gemittelten Röntgenintensität die für die einzelnen Pixelelektroden jeweils erfassten Zählraten als Eingangsgröße für die zeitliche Variation der jeweiligen Depletionsspannung herangezogen.

In einer besonders vorteilhaften Ausführung des Verfahrens wird der funktionelle Zusammenhang zwischen der Bestrahlungshistorie und der ortsabhängig einzustellenden Depletionsspannung mittels eines bestimmten, nachfolgend näher beschriebenen Kalibrierungsprozesses bestimmt. Hierzu wird zunächst der Röntgendetektor in einer Belastungsphase mit homogener Röntgenstrahlung vergleichsweise hoher Intensität bestrahlt. Als "homogene Röntgenstrahlung" wird hierbei Röntgenstrahlung verstanden, deren Intensität und Quantenenergieverteilung (Spektralform) über die Detektorfläche konstant sind. Zweck dieser Belastungsphase ist hierbei eine gezielte Erzeugung einer Pixelgrößendrift auf der Detektorfläche.

In einer an die Belastungsphase anschließenden Messphase wird mehrfach (d.h. zu mindestens zwei unterschiedlichen Messzeitpunkten) unter Bestrahlung des Röntgendetektors mit homogener Röntgenstrahlung für jede Pixelelektrode sowie für mindestens zwei Spektralbereiche jeweils die Zählrate bestimmt. Der einem Zählereignis zugeordnete Spektralbereich wird dabei insbesondere jeweils über die Spitzenstromstärke $H$ des detektierten Strompulses bestimmt, der - wie vorstehend beschrieben - mit der Frequenz (und entsprechend der Quantenenergie) der absorbierten Röntgenstrahlung korreliert. Mit anderen Worten wird für jede Pixelelektrode und jeden Messzeitpunkt jeweils ein Zählratenspektrum ermittelt (der Begriff "Zählratenspektrum" wird hierbei im Sinne einer prägnanten Nomenklatur auch dann verwendet, wenn dieses Zählratenspektrum nur zwei Stützstellen umfasst).

In einem weiteren Schritt des Kalibrierungsprozesses wird für jedes Zählratenspektrum jeweils der niederfrequente Grenzwert bestimmt. Als "niederfrequenter Grenzwert" wird hierbei derjenige Zählratenwert bezeichnet, den das jeweilige Zählratenspektrum in Extrapolation gegen verschwindende Frequenz bzw. Quantenenergie bzw. Spitzenstromstärke (d.h. für $H = 0$) annimmt. Die Stützstellen des jeweiligen Zählratenspektrums werden hierbei vorzugsweise durch eine lineare oder polynominale Modellfunktion angepasst (gefittet), wobei das Zählratenspektrum anhand der angepassten Modellfunktion dann gegen $H = 0$ extrapoliert wird. Dieser niederfrequente Grenzwert wird im Rahmen des Kalibrierungsprozesses als Maß für die Gesamtzahl der von der jeweiligen Pixelelektrode zu dem jeweiligen Messzeitpunkt erfassten Pulse herangezogen, zumal dieser Grenzwert erkanntermaßen von der spektralen Stauchung und den dadurch verursachten Zählfehlern unbeeinflusst bleibt.

Aus mindestens zwei zu unterschiedlichen Messzeitpunkten ermittelten niederfrequenten Grenzwerten wird schließlich im Rahmen des Kalibrierungsprozesses, insbesondere durch Division dieser Grenzwerte, für jede Pixelelektrode ein Maß für die Pixelgrößendrift berechnet. Anhand dieses Maßes für die Pixelgrößendrift wird dann im laufenden Betrieb des Röntgendetektors für die jeweilige Pixelelektrode die zugehörige Depletionsspannung bestimmt.

Die Korrelation zwischen dem Maß für die Pixelgrößendrift und der jeweils einzustellenden Depletionsspannung kann hierbei im Rahmen der Erfindung entweder empirisch, zum Beispiel durch numerische Optimierungsverfahren ermittelt oder anhand der gegebenen Materialkonstanten sowie der geometrischen Eigenschaften des Röntgendetektors berechnet werden.

Die erfindungsgemäße Steuereinheit ist allgemein zur Durchführung des vorstehend beschriebenen, erfindungsgemäßen Verfahrens eingerichtet. Sie ist somit insbesondere dazu eingerichtet, an jede der Pixelelektroden des anzusteuernden Röntgendetektors - insbesondere durch individuelle Einstellung des elektrischen (Betriebs-)Potentials der jeweiligen Pixelelektrode - eine individuell eingestellte Depletionsspannung anzulegen und die an die verschiedenen Pixelelektroden anzulegenden Depletionsspannungen derart verschieden zu wählen, dass die den Pixelelektroden jeweils zugeordneten effektiven Pixelgrößen aneinander angeglichen werden.

Die Steuereinheit ist hierbei insbesondere dazu eingerichtet, das Verfahren in einer der vorstehend beschriebenen Gestaltungsformen auszuführen. Die vorstehenden Ausführungen zu unterschiedlichen Varianten und Weiterbildungen des Verfahrens sind somit sinngemäß auf die Steuereinheit zu übertragen.

Bei der Steuereinheit handelt es sich um eine körperliche Vorrichtung, in der die Funktion zur Durchführung des Verfahrens durch schaltungs- und/oder programmtechnische Mittel implementiert ist. Vorzugsweise umfasst diese Steuereinheit hierbei um einen nicht-programmierbaren integrierten Schaltkreis, insbesondere einen sogenannten ASIC, in dem das Verfahren oder funktionale Bestandteile desselben schaltungstechnisch implementiert sind. Zusätzlich oder alternativ hierzu kann die Steuereinheit im Rahmen der Erfindung allerdings auch ein programmierbares Bauteil, insbesondere ein FPGA (Field Programmable Gate Array) oder einen Mikroprozessor umfassen, in dem das Verfahren oder funktionale Bestandteile desselben durch ein Programm implementiert sind.

Die Steuereinheit kann im Rahmen der Erfindung separat von dem anzusteuernden Röntgendetektor, insbesondere als Zubehörteil oder Nachrüstsatz für einen bestehenden Röntgendetektor hergestellt werden. Die Steuereinheit kann aber auch als fester, insbesondere integrierter Bestandteil eines Röntgendetektors realisiert sein, so dass auch ein mit der Steuereinheit versehener Röntgendetektor eine Verkörperung der Erfindung darstellt.

Nachfolgend werden Ausführungsbeispiele der Erfindung anhand einer Zeichnung näher beschrieben. Darin zeigen:

FIG 1     in einem schematischen Blockschaltbild einen Röntgendetektor mit einer Sensorschicht, an deren Vorderseite eine sich über die gesamte Detektorfläche erstreckende Steuerelektrode angeordnet ist, und an deren Rückseite eine Vielzahl von einzelnen Pixelektroden mit jeweils nachgeschalteter Auswerteelektronik angeordnet sind, sowie ein FPGA zur Ansteuerung des Röntgendetektors,

FIG 2     in einer vergrößerten Detaildarstellung II gemäß FIG 1 einen Ausschnitt der Sensorschicht des Röntgendetektors, anhand welcher die ungestörte Feldkonfiguration eines sich unter Wirkung einer Depletionsspannung im Inneren der Sensorschicht aufbauenden elektrischen Feldes angedeutet ist,

FIG 3     in Darstellung gemäß FIG 2 den dortigen Ausschnitt durch die Sensorschicht mit einer durch Pixelgrößendrift gestörten Feldkonfiguration,

FIG 4     in einem elektrischen Schaltbild einen einer jeden Pixelektrode jeweils beigeordneten Vorverstärker der Auswerteelektronik des Röntgendetektors gemäß FIG 1,

FIG 5     in Darstellung gemäß FIG 4 eine alternative Ausführungsform des Vorverstärkers,

FIG 6     in einem schematischen Schaltbild einen einer jeden Pixelektrode jeweils beigeordneten Spannungsteiler zur variablen Einstellung des Betriebspotentials für die jeweilige Pixelektrode,

FIG 7     in einem schematischen Flussdiagramm ein Verfahren zur Bestimmung und Einstellung der Betriebspotentiale der Pixelektroden, und

FIG 8     in einem Diagramm der Zählrate gegen die Spitzenstromstärke der an einer Pixelektrode detektierten Strompulse zwei im Zuge des Verfahrens gemäß FIG 7 aufgenommene Zählratenspektren.

Einander entsprechende Teile und Größen sind in allen Figuren stets mit gleichen Bezugszeichen versehen.

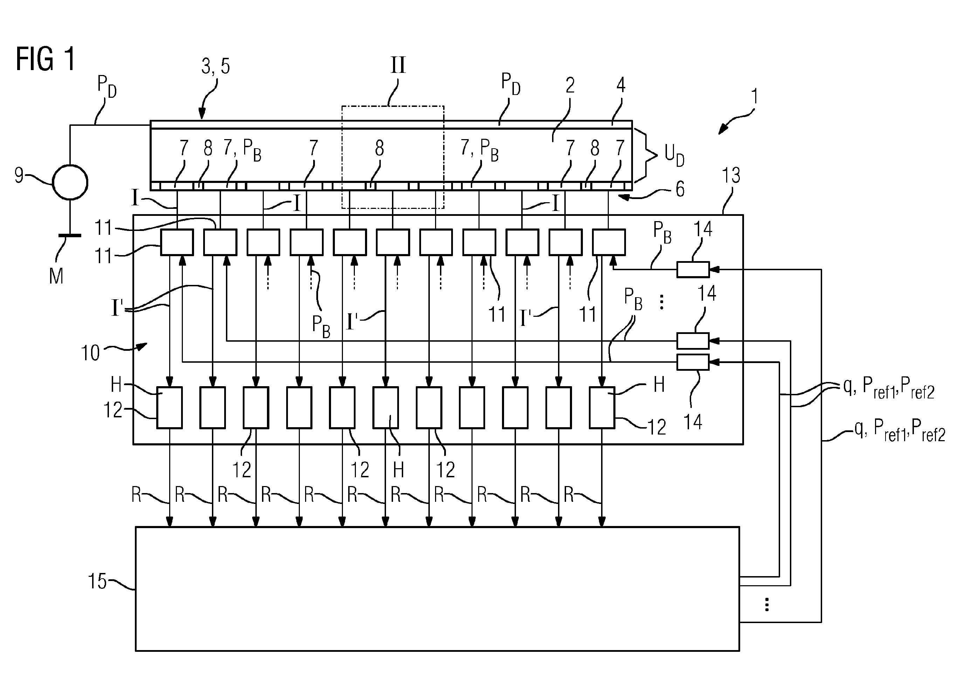

In FIG 1 ist ein Röntgendetektor 1 dargestellt, der beispielsweise in einem medizinischen Computertomographen eingesetzt ist. Bei dem Röntgendetektor 1 handelt es sich um einen direktwandelnden, quantenzählenden Röntgendetektor. Der Röntgendetektor 1 umfasst eine Sensorschicht 2 aus einem röntgensensitiven, halbleitenden Material, insbesondere CdTe (Cadmium Tellurid). Eine Vorderseite 3 der Sensorschicht 2, die in der bestimmungsgemäßen Anordnung des Röntgendetektors 1 im Betrieb einer (nicht näher dargestellten) Röntgenquelle zugeordnet ist, ist mit einer Steuerelektrode 4 versehen, die sich über die gesamte Detektorfläche 5, also die vorderseitige Fläche der Sensorschicht 2 erstreckt. An einer der Vorderseite 3 gegenüberliegenden Rückseite 6 der Sensorschicht 2 sind eine Vielzahl von einzelnen, gegeneinander isolierten Pixelektroden 7 angeordnet. Die Pixelektroden 7 sind hierbei in einer zweidimensionalen, rechteckigen Gitterstruktur (Matrixstruktur) angeordnet und unter Bildung von zwischengeordneten (Elektroden-)Zwischenräumen 8 zueinander beabstandet.

In einer beispielhaften Dimensionierung hat die Sensorschicht 2 eine Stärke von 1600 $\mu$m, einen sogenannten Pixelpitch, d.h. einen Abstand zwischen den Flächenzentren (Mitte-Mitte-Abstand) benachbarter Pixelektroden 7, von jeweils 250 $\mu$m sowie einen Pixelektrodenabstand von jeweils 55 $\mu$m.

Die Steuerelektrode 4 ist mit einer (Hoch-)Spannungsquelle 9 verschaltet. Jeder der Pixelektroden 7 ist andererseits einer Auswerteelektronik 10 nachgeschaltet. Im Rahmen der Auswerteelektronik 10 ist jeder Pixelektrode 7 ein Vorverstärker 11 unmittelbar nachgeschaltet. Desweiteren umfasst die Auswerteelektronik 10 für jede Pixelektrode 7 einen dem Vorverstärker 11 jeweils nachgeschalteten Zählerschaltkreis 12. Die Auswerteelektronik 10 ist im Ausführungsbeispiel gemäß FIG 1 in einem ASIC 13 integriert.

Im Betrieb des Röntgendetektors 1 wird durch die Hochspannungsquelle 9 ein negatives Depletionspotential $P_D$ angelegt, das beispielsweise einen Wert von -900 Volt gegenüber Massenpotential M aufweist. Die Pixelektroden 7 werden andererseits - in nachfolgend in näher beschriebener Weise - auf Betriebspotentialen $P_B$ gehalten, die nur geringfügig, beispielsweise innerhalb eines Intervalls [-10V; +10V] um Massenpotential M (M = 0V) variieren. Somit liegt innerhalb der Sensorschicht 2 zwischen der Steuerelektrode 4 und jeder der Pixelektroden 7 eine Depletionsspannung $U_D$ ($U_D = P_B - P_D$) an, Betrag in der Größenordnung von 900V liegt.

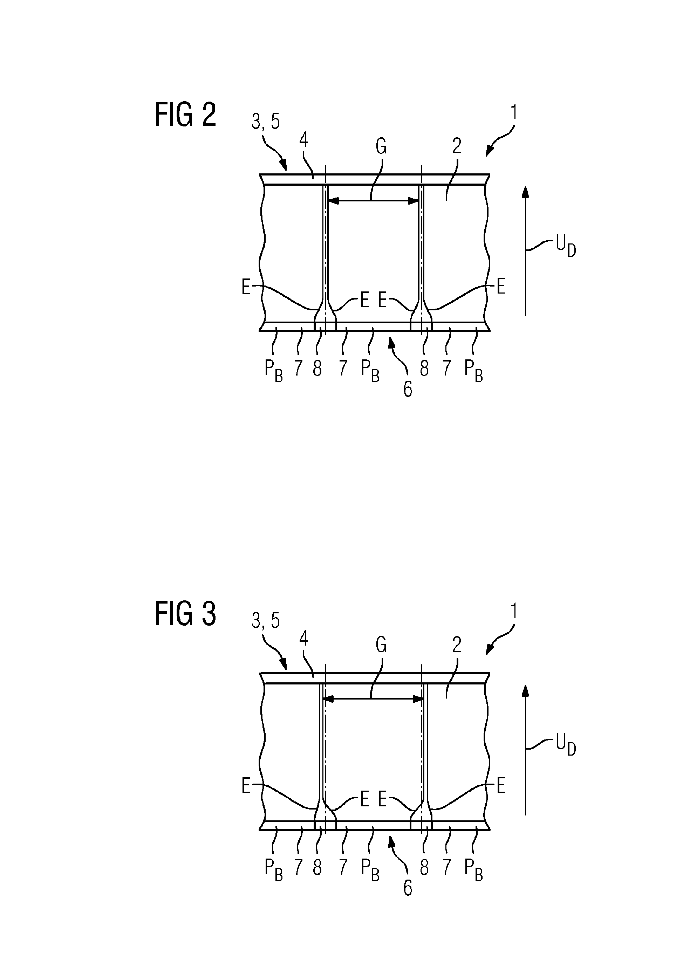

Wie in FIG 2 schematisch dargestellt ist, baut sich unter Wirkung der Depletionsspannung $U_D$ innerhalb der Sensorschicht 2 ein (in FIG 2 lediglich angedeutetes) elektrisches Feld E auf. Der sich zwischen der Steuerelektrode 4 und jeweils einer bestimmten Pixelektrode 7 erstreckende Teil dieses Feldes E definiert hierbei einen Einflussbereich der jeweiligen Pixelektrode 7 insofern, als Elektronen, die durch Absorption von Röntgenquanten in diesem Einflussbereich aus ihrem gebundenen Zustand ausgeschlagen werden, entlang der Feldlinien des Feldes E zu dieser Pixelektrode 7 driften. Die - an oder in der Nähe der Vorderseite 4 der Sensorschicht 2 gemessene - Querschnittsfläche dieses Einflussbereiches bildet eine (effektive) Pixelgröße G. Wie der Darstellung gemäß FIG 2 zu entnehmen ist, übersteigt diese Pixelgröße G zumindest in dem in FIG 2 dargestellten, idealen (ungestörten) Zustand des Röntgendetektors 1 die Querschnittsfläche der zugeordneten Pixelelektrode 7, zumal sich das Feld E, und somit der Einflussbereich der Pixelelektrode 7, in den Bereich der angrenzenden Elektrodenzwischenräume 8 ausbreiten.

In dem ungestörten Zustand des Röntgendetektors 1 gemäß FIG 2 füllen die jeweiligen Einflussbereiche zweier aneinander angrenzender Pixelelektroden 7 den Bereich des dazwischen liegenden Elektrodenzwischenraums 8 zu gleichen Teilen aus. Hierdurch weisen alle Pixelelektroden 7 die betragsmäßig gleiche Pixelgröße G auf.

Wie in FIG 3 verdeutlicht, weichen die Pixelgrößen G der einzelnen Pixelelektroden 7 in der Realität allerdings infolge der Pixelgrößendrift mehr oder weniger stark voneinander ab. Eine Vergrößerung der Pixelgröße G einer bestimmten Pixelelektrode 7 (im Beispiel gemäß FIG 3 der mittleren Pixelelektrode 7) geht hierbei zwangsläufig zu Lasten der Pixelgröße G mindestens einer angrenzenden Pixelelektrode 7. Bekanntermaßen kann eine solche Pixelgrößendrift erzeugt werden, indem die Pixelelektroden 7 auf voneinander abweichende Betriebspotentiale $P_B$ gelegt werden. In Umkehr dieses Vorgehens wird bei dem Röntgendetektor 1 eine bestehende Pixelgrößendrift durch individuelle Einstellung des Betriebspotentials $P_B$ für jede Pixelelektrode ausgeglichen. Die bei Absorption eines Röntgenquants ausgeschlagenen Elektronen lösen in der Pixelelektrode 7, in deren Einflussbereich die Absorption stattgefunden hat, gemäß FIG 1 einen Strompuls I aus. Dieser Strompuls I wird durch den nachgeschalteten Vorverstärker 11 in einen stromproportionalen Spannungspuls I' gewandelt. Dieser Spannungspuls I' wird von dem Vorverstärker 11 dem nachgeschalteten Zählerschaltkreis 12 zugeführt und führt hier zu einem Zählereignis. In dem Zählerschaltkreis 12 wird der Spitzenstromwert H des Strompulses I (genauer gesagt der Spitzenwert des stromproportionalen Spannungspulses I') mit einer Kaskade von mehreren - beispielsweise vier - abgestuften Schwellwerten verglichen. Sofern der Spitzenstromwert H hierbei eine vorgegebene Mindesthöhe erreicht, wird der Strompuls I, je nach Höhe des Spitzenstromwerts H, in einem von mehreren Kanälen als Zählereignis gezählt. Somit werden die Zählereignisse spektral aufgelöst, also in Abhängigkeit der Frequenz oder Quantenenergie des detektierten Röntgenquants erfasst.

Die vorstehend beschriebene Pixelgrößendrift führt hierbei - bei konstanter Intensität der einfallenden Röntgenstrahlung - zu einer Änderung der Zählrate, die proportional zu der Änderung der effektiven Pixelgröße G ist. Um die Pixelgrößendrift zu eliminieren oder zu mindestens weitgehend zu reduzieren, werden die Betriebspotentiale $P_B$ für jede Pixelelektrode 7 mittels eines jeweils zugeordneten Anpassungsschaltkreises 14 (FIG 1) individuell eingestellt. Die Pixelelektroden 7 werden somit in der Regel auf geringfügig verschiedenen Betriebspotentialen $P_B$ gehalten, wodurch auch die Depletionsspannung $U_D$ für die verschiedenen Pixelelektroden 7 geringfügig verschieden ist. Das Betriebspotential $P_B$ wird hierbei der jeweiligen Pixelelektrode 7 über den zugeordneten Vorverstärker 11 zugeführt.

Die - im Beispiel gemäß FIG 1 ebenfalls in dem ASIC 13 integrierten - Anpassungsschaltkreise 14 werden andererseits von einem FPGA 15 (d.h. einem Field Programmable Gate Array) mit einer - für alle Anpassungsschaltkreise 14 einheitlichen Stellgröße q angesteuert.

Der FPGA 15 ist der Auswerteelektronik 10 nachgeschaltet und erhält von dieser als Eingangsgrößen die von dem Zählerschaltkreis 12 ausgegebenen Zählraten R für die einzelnen Pixelelektroden 7. Der FPGA 15 berechnet hieraus auf nachstehend näher beschriebener Weise das Stellsignal q für die Anpassungsschaltkreise 14.

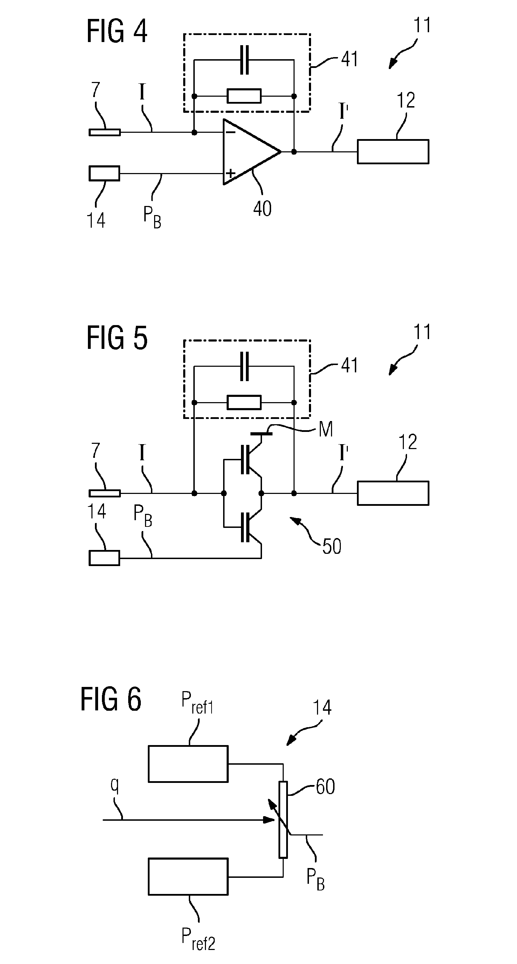

In FIG 4 ist eine beispielhafte Ausführung eines der Vorverstärker 11 abgebildet. Der Vorverstärker 11 ist hierbei als Transimpedanzverstärker ausgebildet. Der Vorverstärker 11 umfasst in dieser Ausführungsform einen Operationsverstärker 40, dessen invertierender Eingang (-) über einen Rückkopplungsschaltkreis 41 mit dem Ausgang rückgekoppelt ist. Der invertierende Eingang des Operationsverstärkers 40 ist mit der zugeordneten Pixelelektrode 7 verbunden. Der nichtinvertierende Eingang (+) des Operationsverstärkers 21 ist dagegen zur Zuführung des Betriebspotentials $P_B$ mit dem Anpassungsschaltkreis 14 verbunden. Der Ausgang des Operationsverstärkers 40 ist mit dem zugeordneten Zählerschaltkreis 12 verbunden.

FIG 5 zeigt eine alternative Ausführungsform des Vorverstärkers 11. Diese Ausführungsform des Vorverstärkers 11 unterscheidet sich von der Ausführungsform gemäß FIG 4 dadurch, dass der Operationsverstärker 40 durch einen komplementären Metall-Oxid-Halbleiter (CMOS 50) ersetzt ist. Die Pixelelektrode 7 ist hierbei mit dem Eingang des CMOS 50 verschaltet, während der Zählerschaltkreis 12 mit dem Ausgang des CMOS 50 verschaltet ist. Der Anpassungsschaltkreis 14 ist zur Zuführung des Betriebspotentials $P_B$ mit dem negativen Versorgungsspannungsanschluss (VSS) des CMOS 50 verbunden.

Das von dem FPGA 15 an die Anpassungsschaltkreis 14 ausgegebene Stellsignal q kann - kontinuierlich oder diskontinuierlich in mehreren Stufen - zwischen zwei Grenzwerten, z.B. zwischen 0 und 1, variieren. Der jeweilige Anpassungsschaltkreis 14 variiert das Betriebspotential $P_B$ für die zugehörige Pixelelektrode 7 hierbei insbesondere gemäß einer linearen Beziehung

$$P_B = q \cdot P_{ref\,1} + \left(1 - q\right) \cdot P_{ref\,2} \qquad\qquad \text{GLG 1}$$

zwischen zwei vorgegebenen Referenzpotentialen $P_{ref1}$ und $P_{ref2}$.

Jeder der Anpassungsschaltkreise 14 ist hierbei gemäß FIG 6 insbesondere durch einen Spannungsteiler 60 gebildet, an dem die - für jede Pixelelektrode 7 individuell festgelegten - Referenzpotentiale $P_{ref1}$ und $P_{ref2}$ angelegt sind, und dessen Teilungsverhältnis automatisch nach Maßgabe der Stellgröße q variierbar ist.

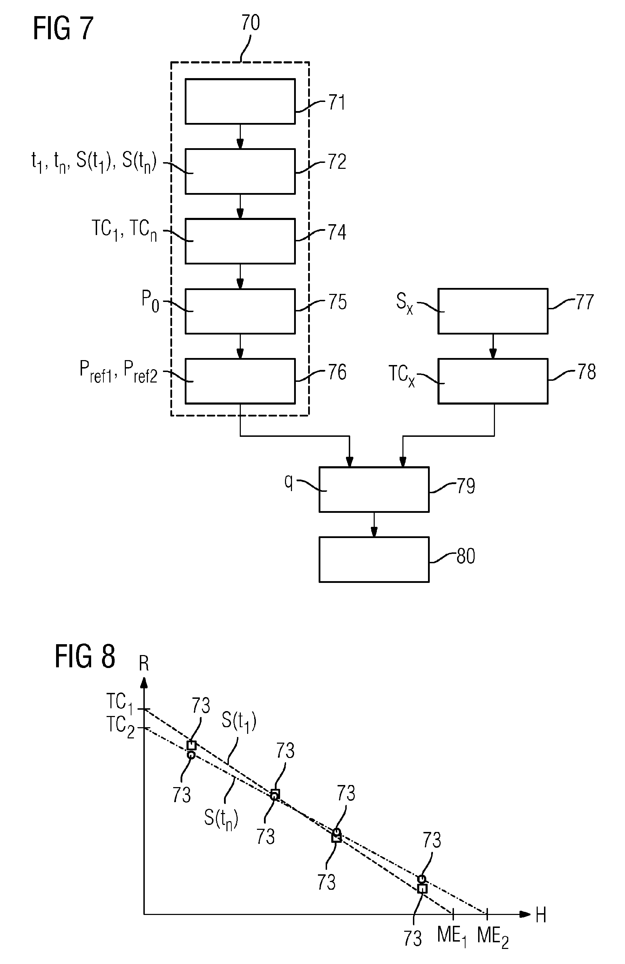

Die Referenzpotentiale $P_{ref1}$, $P_{ref2}$ und die Stellgröße q werden mittels des FPGA 15 nach einem in FIG 7 näher dargestellten Verfahren ermittelt.

Dem laufenden Betrieb des Röntgendetektors 1, also dem bestimmungsgemäßen Betrieb des Röntgendetektors 1 zur Aufnahme von medizinischen Röntgenbildern, ist hierbei ein Kalibrierungsprozess 70 vorgeschaltet. Im Rahmen dieses Kalibrierungsprozesses 70 wird der Röntgendetektor 1 zunächst während einer Belastungsphase 71 für eine vorgegebene Zeitdauer mit homogener Röntgenstrahlung einer vergleichsweise hohen Intensität bestrahlt. Die Röntgenstrahlung ist hierbei hinsichtlich Dauer und Intensität derart bemessen, dass die dadurch verursachte Belastung des Röntgendetektors 1 etwa der im laufenden Betrieb des Röntgendetektors 1 zur erwartenden Maximalbelastung entspricht.

An die Belastungsphase 71 schließt eine Messphase 72 an, in der wiederholt unter Bestrahlung des Röntgendetektors 1 mit homogener Röntgenstrahlung für jede Pixelelektrode 7 spektralaufgelöst die Zählraten R bestimmt werden. In der Messphase 72 werden hierbei zumindest eine erste Messung zu einem Zeitpunkt $t_1$ unmittelbar nach dem Ende der Belastungsphase 71 sowie eine zweite Messung einem Zeitpunkt $t_n$ am Ende der Messphase 72 durchgeführt. Die Dauer der Messphase 72, und somit der zeitliche Abstand zwischen den Zeitpunkten $t_1$ und $t_n$ ist hierbei derart gewählt, dass sie etwa der zu erwartenden Regenerierungsphase der Sensorschicht 2 nach der Belastungsphase 71 entspricht. Zum Messzeitpunkt $t_1$ werden somit die Zählraten R bei vollbelastetem Röntgendetektor 1 bestimmt, während zum Messzeitpunkt $t_n$ die Zählraten R bei regeneriertem Röntgendetektor 1 gemessen werden.

Während der Messphase 72 wird der Röntgendetektor 1 lediglich diskontinuierlich zu den Messzeitpunkten $t_1$ und $t_n$ mit Röntgenstrahlung bestrahlt, wobei diese Röntgenstrahlung zu beiden Messzeitpunkten $t_1$ und $t_n$ die gleiche, im Vergleich zu der während der Belastungsphase 71 applizierten Röntgenstrahlung wesentlich niedrigere Intensität aufweist.

Entsprechend der spektralen Auflösung der Zählerschaltkreise 12 liefert die Auswerteelektronik 10 an den FPGA 15 für jede Pixelelektrode 7 und zu jedem der beiden Messzeitpunkte $t_1$ und $t_n$ jeweils ein Tupel von vier Zählratenwerten, wobei jeder Zählratenwert - wie vorstehend beschrieben - einem bestimmten Spektralbereich (und entsprechend einer bestimmten Spitzenstromstärke H) zugeordnet ist. Jeder dieser Zählratenwerte kann somit als Stützstelle 73 (FIG 8) eines Zählratenspektrums $S(t_1)$ bzw. $S(t_n)$ dargestellt werden. Ein schematisches Beispiel für die beiden, für eine bestimmte Pixelelektrode 7 zu den Messzeitpunkten $t_1$ bzw. $t_n$ aufgenommenen Zählratenspektrums $S(t_1)$ und $S(t_n)$ ist in FIG 8 dargestellt. Die Stützstellen 73 des Spektrums $S(t_1)$ sind hierbei mit Rechtecksymbolen dargestellt, während die Stützstellen 73 des Zählratenspektrums $S(t_n)$ mit Kreissymbolen dargestellt sind.

FIG 8 ist zu entnehmen, dass der Wert der Zählratenspektren $S(t_1)$ und $S(t_n)$ monoton mit steigender Spitzenstromstärke H (und entsprechend steigender Quantenenergie) abnimmt. Die mittels des Röntgendetektors 1 detektierbaren Zählratenspektren $S(t_1)$ und $S(t_n)$ unterscheiden sich dabei insofern von dem Spektrum der einfallenden Röntgenstrahlung, als die Zählratenspektren regelmäßig zu niedrigeren Energiewerten verschoben sind. Dies liegt einerseits an der eingangs beschriebenen Spektralstauchung sowie andererseits daran, dass die durch ein Röntgenquant erzeugte Ladungsmenge mitunter auf zwei oder mehr benachbarte Pixelelektroden 7 aufgeteilt wird, wodurch auch kleinere Energiemengen, die die Quantenenergien absorbierten Röntgenquanten deutlich unterschreiten, von den Pixelelektroden 7 detektiert werden.

In einem auf die Messphase 72 folgenden Verfahrensschritt 74 (FIG 7) wird nun durch den FPGA 15 jedes der beiden Zählratenspektren $S(t_1)$ und $S(t_n)$ linear angepasst (gefittet) . Anhand dieser Anpassung bestimmt der FPGA 15 für jedes der Zählratenspektren $S(t_1)$ und $S(t_n)$ den jeweiligen niederenergetischen Grenzwert $TC_1$ bzw. $TC_n$ (FIG 8). Als "niederenergetischer Grenzwert" $TC_1$ bzw. $TC_n$ wird hierbei der Zählratenwert bezeichnet, den das jeweilige Zählratenspektrum $S(t_1)$ bzw. $S(t_n)$ in der Extrapolation zu verschwindenden Energien (H = 0) annimmt ( $TC_1 = S(t_1)|_{H=0}$; $TC_n = S(t_n)|_{H=0}$) . Der so bestimmte Grenzwert $TC_1$ bzw. $TC_n$ wird im Rahmen des Verfahrens als Maß für die Gesamtzahl der im Einflussbereich der jeweiligen Pixelelektrode 7 zum jeweiligen Messzeitpunkt $t_1$ bzw. $t_n$ absorbierten Röntgenquanten herangezogen, da dieser Grenzwert $TC_1$ bzw. $TC_n$ von der spektralen Stauchung weitgehend unbeeinflusst bleibt.

Bekanntermaßen äußert sich die spektrale Stauchung hingegen in den Zählratenspektren $S(t_1)$ und $S(t_n)$ maßgeblich in der Lage der jeweiligen Nullstelle $ME_1$ bzw. $ME_n$ (FIG 8).

In einem anschließenden Verfahrensschritt 75 (FIG 7) des Kalibrierungsprozesses 70 bestimmt der FPGA 15 für jede Pixelelektrode 7 aus den zuvor bestimmten Grenzwerten $TC_1$ und $TC_n$ ein Maß für die Pixeldrift, das nachfolgend als Pixeldriftstärke $P_0$ bezeichnet ist:

$$P_0 = \frac{TC_1}{TC_n} \qquad\qquad \text{GLG 2}$$

Die für die einzelnen Pixelelektroden 7 ermittelten Pixeldriftstärken $P_0$ ergeben hierbei ein Pixeldriftmuster, das die räumliche Verteilung der effektiven Pixelgröße G im vollbelasteten Zustand des Röntgendetektors 1 wiederspiegelt. Die für die Pixelelektroden 7 ermittelten Pixeldriftstärken $P_0$ werden in dem FPGA 15 zur Verwendung im laufenden Betrieb des Röntgendetektors 1 beispielsweise in Form eines Vektors hinterlegt, der für jede Pixelelektrode 7 als Vektoreintrag $[P_0]_i$ die zugehörige Pixeldriftstärke $P_0$ enthält. Die Größe i (mit i = 1, 2,...,m) bezeichnet hierbei eine Zählvariable, die die jeweilige Pixelelektrode 7 kennzeichnet.

In einem Verfahrensschritt 76 bestimmt der FPGA 15 für jede Pixelelektrode 7 Werte für die Referenzpotentiale $P_{ref1}$ und Pref2.

Das Referenzpotential $P_{ref2}$ ist hierbei dem unbelasteten Zustand des Röntgendetektors 1 zugeordnet, in dem die Pixelgrößendrift nur schwach ausgeprägt ist. Dementsprechend kann das Referenzpotential $P_{ref2}$ in einfacher Ausprägung des Verfahrens für alle Pixelelektroden 7 konstant (insbesondere mit dem Wert $P_{ref2} = 0$) vorgegeben werden. Vorzugsweise ist aber vorgesehen, dass der FPGA 15 das Referenzpotential $P_{ref2}$ anhand der für den Messzeitpunkt $t_n$ für die einzelnen Pixelelektroden 7 ermittelten Grenzwerte $TC_n$ bestimmt. Der FPGA 15 bestimmt die Referenzpotentiale $P_{ref2}$ für die einzelnen Pixelelektroden 7 hierbei derart, dass die räumlichen Unterschiede zwischen diesen Grenzwerten $TC_n$ kompensiert werden, zum Beispiel gemäß

$$P_{ref2} = -C_1 \cdot \left( TC_n \right) \qquad \text{GLG 3}$$

Das Referenzpotential $P_{ref1}$ wird von dem FPGA 15 gemäß einer hinterlegten funktionalen Abhängigkeit von der Pixeldriftstärke $P_0$ bestimmt, insbesondere nach der Beziehung

$$P_{ref1} = P_{ref2} - C_2 \cdot P_0 \qquad \text{GLG 4}$$

Die Größen $C_1$ und $C_2$ in den GLG 3 und 4 bezeichnen Proportionalitätskonstanten, die durch empirische Versuche, insbesondere die Anwendung eines numerischen Optimierungsverfahrens bestimmt oder anhand der Materialeigenschaften und geometrischen Abmessungen der Sensorschicht 2 und der Pixelelektroden 7 berechnet werden.

Die nach GLG 3 oder 4 berechneten Werte der Referenzpotentiale $P_{ref1}$ und $P_{ref2}$ werden durch den FPGA 15 an die Anpassungsschaltkreise 14 übermittelt und dort hinterlegt.

Im laufenden Betrieb des Röntgendetektors 1 wird in einem Verfahrensschritt 77 mittels des Röntgendetektors 1 ein Röntgenbild aufgenommen. Zu jedem Pixel dieses Röntgenbildes wird hierbei analog zu dem Vorgehen in der Messphase 72 ein Zählratenspektrum $S_x$ bestimmt. Durch - wiederum lineare - Anpassung dieses Zählratenspektrums $S_x$ und Extrapolation des angepassten Spektrums gegen verschwindende Energie (H = 0) wird aus diesem Zählratenspektrum $S_x$ in einem Verfahrensschritt 78 für jede Pixelelektrode 7 der niederenergetische Grenzwert $TC_x$ bestimmt. Die für die einzelnen Pixelelektroden 7 ermittelten Grenzwerte $TC_x$ werden dabei durch den FPGA 15 beispielsweise zu einem Vektor mit Vektoreinträgen $[TC_x]_i$ (mit i = 1, 2,..., m) zusammengefasst, wobei die Zählvariable i wiederum die jeweilige Pixelelektrode 7 kennzeichnet.

In einem Verfahrensschritt 79 bestimmt der FPGA 15 durch Skalarmultiplikation der Vektoreinträge $[P_0]_i$ und $[TC_x]_i$ die Stellgröße q:

$$q = A \cdot \sum_{i=1}^{m} \left( [P_0]_i \cdot [TC_x]_i \right) - B \qquad \text{GLG 5}$$

Die Größen A und B sind Normierungskonstanten, die derart gewählt sind, dass die Stellgröße q im Grenzfall $TC_x = TC_1$ den Wert 1, und im Grenzfall $TC_x = TC_n$ den Wert 0 annimmt.

Da das aufgenommene Röntgenbild durch die unbekannte, variable Struktur des untersuchten Objekts beeinflusst wird, kann das Skalarprodukt deutlichen Schwankungen unterliegen. Um diese Schwankungen abzumildern, wird das Skalarprodukt vorzugsweise durch Filterung (z.B. Mittelung) geglättet. Zusätzlich oder alternativ hierzu kann die Bildung des Skalarprodukts numerisch vereinfacht werden, indem nicht alle Pixel, sondern nur ausgesuchte Pixel berücksichtigt werden, dabei bevorzugt randseitige Pixel, die mit nur geringer Wahrscheinlichkeit von dem zu untersuchenden Objekt beeinflusst werden.

In einem abschließenden Verfahrensschritt 80 übermittelt der FPGA 15 die Stellgröße q an die Anpassungsschaltkreise 14, die wiederum nach GLG 1 für die jeweils zugeordnete Pixelelektrode 7 das Betriebspotential $P_B$ einstellen.

In dem dargestellten Ausführungsbeispiel wird die erfindungsgemäße Steuereinheit durch die Kombination des ASIC 13 und des FPGA 15 gebildet.

Durch die unterschiedlichen Betriebspotentiale $P_B$ werden Unterschiede der effektiven Pixelgröße G der einzelnen Pixelelektroden 7 dynamisch ausgeglichen.

In einer (nicht näher dargestellten) Variante des Verfahrens wird mittels des FPGA 15 das zeitliche Verhalten beim Aufbau sowie dem Abklingen einer belastungsbedingten Pixelgrößendrift bestimmt.

Hierzu wird der Röntgendetektor 1 im Rahmen eines modifizierten Kalibrierungsprozesses in mehreren Belastungsphasen mit unterschiedlicher Röntgenintensität und/oder Bestrahlungsdauer bestrahlt, wobei in einer an jede Belastungsphase jeweils anschließende Messphase analog zu FIG 7 die Pixeldriftstärke $P_0$ bestimmt wird. Die Abhängigkeit der Pixeldriftstärke $P_0$ von der Intensität RI und Dauer RT der Bestrahlung wird hierbei vorzugsweise durch ein exponentielles Anreicherungsmodell mit angepasster Zeitkonstante $\tau1$ modelliert:

$$P_0\left(RI, RT\right) = P_0 * \cdot\left(1 - e^{-t/\tau1}\right) \qquad \text{GLG 6}$$

Des Weiteren werden in den Messphasen jeweils mindestens drei Messungen zu Messzeitpunkten $t_1$, $t_2$, ..., $t_n$ durchführt, mittels welcher das Abklingverhalten durch ein exponentielles Abklingmodell mit angepasster Zeitkonstante $\tau2$ modelliert wird:

$$P\left(t\right) = P_0\left(RI, RT\right) \cdot e^{-1/\tau2} \qquad \text{GLG 7}$$

Die entsprechenden Parameter werden durch den FPGA 15 für den laufenden Betrieb hinterlegt.

Im laufenden Betrieb des Röntgendetektors 1 zeichnet der FPGA 5 hierbei während der Bildaufnahme den Verlauf der auf den Röntgenintensität auf. Anhand der Dauer der Bildaufnahme und der zeitlich gemittelten Röntgenintensität bestimmt der FPGA 15 für jede Pixelelektrode 7 die belastungsabhängige Pixeldriftstärke $P_0$ (RI,RT) gemäß GLG 6. Zu Beginn der folgenden Bildaufnahme bestimmt der FPGA 15 nach GLG 7 anhand der während der Aufnahmepause verstrichenen Zeit die verbleibende Pixelgrößendrift P(t). Der FPGA 15 bestimmt hierbei nach Maßgabe der verbleibenden Pixelgrößendrift P(t) analog zu GLG 4 den Wert des jeweils einzustellenden Betriebspotentials $P_B$ und steuert die Anpassungsschaltkreise 14 entsprechend an.

Die Erfindung wird an dem vorstehend beschriebenen Ausführungsbeispielen besonders deutlich, ist gleichwohl auf diese aber nicht beschränkt. Vielmehr können zahlreiche weitere Ausführungsvarianten der Erfindung aus den Ansprüchen und der vorstehenden Beschreibung abgeleitet werden. Insbesondere können die anhand der Ausführungsbeispiele beschriebenen Einzelmerkmale der Erfindung auch in anderer Weise miteinander kombiniert werden, ohne den Rahmen der Erfindung zu verlassen.

**Patentansprüche**

1. Verfahren zur Ansteuerung eines Röntgendetektors (1), der eine röntgensensitive Sensorschicht (2) sowie eine Anordnung von mit der Sensorschicht (2) rückseitig verbundenen Pixelelektroden (7) umfasst, wobei verfahrensgemäß an jede der Pixelelektroden (7) eine individuell eingestellte Depletionsspannung ($U_D$) angelegt wird, und wobei die an verschiedene Pixelelektroden (7) angelegte Depletionsspannungen ($U_D$) dem Betrag nach derart unterschiedlich gewählt werden, dass die den Pixelelektroden (7) jeweils zugeordneten effektiven Pixelgrößen (G) aneinander angeglichen werden.

2. Verfahren nach Anspruch 1,
   wobei zur Einstellung der Depletionsspannungen ($U_D$) für jede der Pixelelektroden (7) das zugehörige elektrische Potential ($P_B$) individuell eingestellt wird.

3. Verfahren nach Anspruch 1 oder 2,
   wobei die an jede der Pixelelektroden (7) jeweils angelegte Depletionsspannung ($U_D$) in Abhängigkeit der mittels des Röntgendetektors (1) erfassten Röntgenstrahlung zeitlich variiert wird.

4. Verfahren nach Anspruch 3,
   wobei der zeitliche Verlauf der Intensität der erfassten Röntgenstrahlung bei der Einstellung der Depletionsspannungen ($U_D$) berücksichtigt wird.

5. Verfahren nach Anspruch 3 oder 4,

wobei die an jede der Pixelelektroden (7) jeweils angelegte Depletionsspannung ($U_D$) in Abhängigkeit der gemittelten Intensität der erfassten Röntgenstrahlung zeitlich variiert wird.

6. Verfahren nach Anspruch 3,
   wobei die an jede der Pixelelektroden (7) jeweils angelegte Depletionsspannung ($U_D$) in Abhängigkeit der mittels der einzelnen Pixelelektroden (7) erfassten Zählraten (R) zeitlich variiert wird.

7. Verfahren nach einem der Ansprüche 1 bis 6,
   wobei im Rahmen eines Kalibrierungsprozesses (70)

   - der Röntgendetektor (1) in einer Belastungsphase (71) mit homogener Röntgenstrahlung vergleichsweise hoher Intensität bestrahlt wird,
   - nach der Belastungsphase (71) zu mindestens zwei Messzeitpunkten ($t_1$, t2, $t_n$) unter Bestrahlung des Röntgendetektors mit homogener Röntgenstrahlung niedrigerer Intensität für jede Pixelelektrode (7) ein aus mindestens zwei Spektralwerten bestehendes Zählratenspektrum ($S(t_1)$, $S(t_n)$) bestimmt wird,
   - für jede Pixelelektrode (7) und jeden Messzeitpunkt ($t_1$, t2, $t_n$) ein niederfrequenter Grenzwert ($TC_1$, $TC_n$) des jeweiligen Zählratenspektrums ($SC(t_1)$, $SC(t_2)$) ermittelt wird,
   - für jede Pixelelektrode (7) aus mindestens zwei zu unterschiedlichen Messzeitpunkten ($t_1$,t2,$t_n$) ermittelten niederfrequenten Grenzwerten ($TC_1$, $TC_n$) ein Maß ($P_0$) für die Drift der effektiven Pixelgröße (G) berechnet wird, und

   wobei anhand des Maßes ($P_0$) für die Drift der effektiven Pixelgröße (G) für die jeweilige Pixelelektrode (7) die Depletionsspannung ($U_D$) bestimmt wird.

8. Steuereinheit (15) zur Ansteuerung eines Röntgendetektors (1), der eine röntgensensitive Sensorschicht (2) sowie eine Anordnung von mit der Sensorschicht (2) rückseitig verbundenen Pixelelektroden (7) umfasst, wobei die Steuereinheit (15) dazu eingerichtet ist, an jede der Pixelelektroden (7), insbesondere durch individuelle Einstellung des elektrischen Potentials ($U_B$) einer jeden Pixelelektrode (7), eine individuell eingestellte Depletionsspannung ($U_D$) anzulegen, und die an verschiedene Pixelelektroden (7) anzulegenden Depletionsspannungen ($U_D$) dem Betrag nach derart unterschiedlich zu wählen, dass die den Pixelelektroden (7) jeweils zugeordneten effektiven Pixelgrößen (G) aneinander angeglichen werden.

9. Steuereinheit (15) nach Anspruch 8,
   die dazu eingerichtet ist, die an jede der Pixelelektroden (7) jeweils anzulegende Depletionsspannung ($U_D$) in Abhängigkeit der mittels des Röntgendetektors (1) erfassten Röntgenstrahlung zeitlich zu variieren.

10. Steuereinheit (15) nach Anspruch 9,
    die dazu eingerichtet ist, den zeitlichen Verlauf der Intensität der erfassten Röntgenstrahlung bei der Einstellung der Depletionsspannungen ($U_D$) zu berücksichtigen.

11. Steuereinheit (15) nach Anspruch 9 oder 10,
    die dazu eingerichtet ist, die an jede der Pixelelektroden (7) jeweils anzulegende Depletionsspannung ($U_D$) in Abhängigkeit der gemittelten Intensität der erfassten Röntgenstrahlung zeitlich zu variieren.

12. Steuereinheit (1) nach Anspruch 9,
    die dazu eingerichtet ist, die an jede der Pixelelektroden (7) jeweils anzulegende Depletionsspannung ($U_D$) in Abhängigkeit der mittels der einzelnen Pixelelektroden (7) erfassten Zählraten (R) zeitlich zu variieren.

13. Röntgendetektor (15) mit einer röntgensensitiven Sensorschicht (2), mit einer Anordnung von mit der Sensorschicht (2) rückseitig verbundenen Pixelelektroden (7) sowie mit einer Steuereinheit (15) nach einem der Ansprüche 8 bis 12.

FIG 1

EP 2 916 146 A1

FIG 2

FIG 3

FIG 4

FIG 5

FIG 6

## FIG 7

## FIG 8

Europäisches
Patentamt

European
Patent Office

Office européen
des brevets

**EUROPÄISCHER RECHERCHENBERICHT**

Nummer der Anmeldung

EP 15 15 6494

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2007/290142 A1 (DU YANFENG [US] ET AL) 20. Dezember 2007 (2007-12-20) | 1-3,8,13 | INV. G01T1/24 |
| Y | * Absätze [0033] - [0035], [0038], [0048], [0052]; Abbildungen 2,4-12 * | 4-7,9-12 | G01T1/40 |
| | ----- | | |
| X | EP 2 584 379 A1 (BERTHOLD TECHNOLOGIES GMBH & CO KG [DE]) 24. April 2013 (2013-04-24) | 1-3,8 | |
| Y | * Absätze [0016], [0019] - [0021], [0025], [0029] - [0034], [0037], [0038]; Abbildungen 1,2 * | 4-7,9-13 | |
| | ----- | | |
| Y | WO 2014/019818 A1 (SIEMENS AG [DE]) 6. Februar 2014 (2014-02-06) * Seite 2, Zeilen 36-38; Abbildungen 1,7-9 * | 4-6,9-12 | |
| | * Seite 27, Zeile 31 - Seite 28, Zeile 18 * | | |
| | * Seite 45, Zeilen 32-37 * * Seite 47, Zeile 32 - Seite 49, Zeile 13 * | | RECHERCHIERTE SACHGEBIETE (IPC) |
| | ----- | | |
| Y | DE 10 2011 076781 A1 (SIEMENS AG [DE]) 6. Dezember 2012 (2012-12-06) * Absätze [0001], [0002], [0005], [0018] - [0021], [0025], [0028], [0037], [0038]; Abbildungen 1,2 * | 7,13 | G01T |
| | ----- | | |

Der vorliegende Recherchenbericht wurde für alle Patentansprüche erstellt

| Recherchenort | Abschlußdatum der Recherche | Prüfer |
|---|---|---|
| Den Haag | 17. Juli 2015 | Van Ouytsel, Krist'l |

EPO FORM 1503 03.82 (P04C03)

**ANHANG ZUM EUROPÄISCHEN RECHERCHENBERICHT
ÜBER DIE EUROPÄISCHE PATENTANMELDUNG NR.** EP 15 15 6494

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-07-2015

| Im Recherchenbericht angeführtes Patentdokument | Datum der Veröffentlichung | Mitglied(er) der Patentfamilie | Datum der Veröffentlichung |
|---|---|---|---|
| US 2007290142 A1 | 20-12-2007 | JP 2007333734 A<br>US 2007290142 A1 | 27-12-2007<br>20-12-2007 |
| EP 2584379 A1 | 24-04-2013 | KEINE | |
| WO 2014019818 A1 | 06-02-2014 | CN 104641256 A<br>DE 102012213494 A1<br>EP 2864814 A1<br>KR 20150032754 A<br>WO 2014019818 A1 | 20-05-2015<br>06-02-2014<br>29-04-2015<br>27-03-2015<br>06-02-2014 |
| DE 102011076781 A1 | 06-12-2012 | CN 102809756 A<br>DE 102011076781 A1<br>US 2012305757 A1 | 05-12-2012<br>06-12-2012<br>06-12-2012 |

EPO FORM P0461

Für nähere Einzelheiten zu diesem Anhang : siehe Amtsblatt des Europäischen Patentamts, Nr.12/82